# EUROPEAN PATENT SPECIFICATION

(11) **EP 2 141 151 B1**
(45) Date of publication and mention of the grant of the patent: **20.03.2013**
(21) Application number: 08739704.8
(22) Date of filing: 02.04.2008
(51) Int. Cl.: C07D 233/68, C07D 233/90, C07D 233/92

(54) **METHOD FOR PRODUCING 2-HALOIMIDAZOLE COMPOUND**
VERFAHREN ZUR HERSTELLUNG EINER 2-HALOIMIDAZOL-VERBINDUNG
PROCÉDÉ POUR PRODUIRE UN COMPOSÉ DE 2-HALOIMIDAZOLE

(30) Priority: 03.04.2007 JP 2007097005; 21.03.2008 JP 2008073511
(43) Date of publication of application: 06.01.2010
(73) Proprietor: SHIKOKU CHEMICALS CORPORATION, Kagawa 763-8504 (JP)
(72) Inventor: KUMANO, Takeshi, Kagawa 769-0202 (JP); IKEDA, Yuichi, Kagawa 769-0202 (JP)
(74) Representative: Isarpatent
(86) International application number: PCT/JP2008/056593
(87) International publication number: WO 2008/120800

(56) References cited:
- JP-A- 2004 269 500
- JP-A- 2006 117 628
- JAIN R. ET AL.: 'Synthesis of Ring-Halogenated Histidines and Histamines' TETRAHEDRON vol. 54, no. 13, 1998, pages 3235 - 3242, XP004109100
- EVANS D.A. ET AL.: 'Selective Pd0-Mediated C-C Bond Constructions on the Imidazole Ring of L-Histidine: A Practical Approach to the Synthesis of Diphthamide and Related Histidine Analogues' ANGEW. CHEM. INT. ED. ENGL. vol. 32, no. 9, 1993, pages 1326 - 1327, XP008117137

## Description

### Technical Field

The present invention relates to a process for producing a 2-haloimidazole compound useful as an intermediate for pharmaceuticals and agricultural chemicals, an intermediate for dyes, and the like.

### Background Art

In Patent Document 1, as a process for producing a 2-haloimidazole compound, a synthetic method represented by the reaction scheme (A) of chem 1 is disclosed. In Patent Document 2, a synthetic method represented by the reaction scheme (B) of chem 2 is disclosed. In Patent Document 3, a synthetic method represented by the reaction scheme (C) of chem 3 is disclosed.

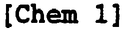

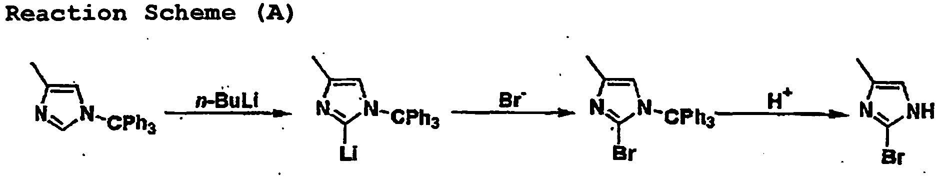

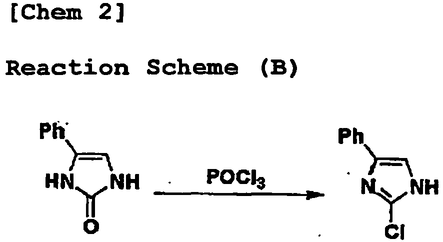

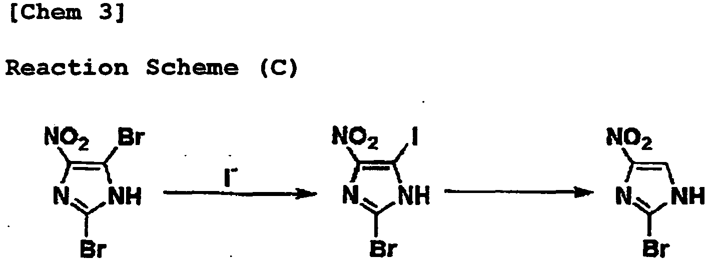

However, in the synthetic method represented by the reaction scheme (A), there is a problem that n-BuLi which must be handled with care should be employed. In the synthetic method represented by the reaction scheme (B), there is a problem that the 2-imidazolone compound to be used as a raw material is expensive and further the reaction yield of the 2-chloroimidazole compound as a product is low. In the synthetic method represented by the reaction scheme (C), there is a drawback that an expensive catalyst such as platinum or palladium-based one should be used in the case of using a hydrogen gas as a reducing agent for an iodinated imidazole compound as an intermediate. Moreover, in the case where a sulfite salt is used instead of the above hydrogen gas, there is a problem that the separation and isolation of the 2-bromoimidazole compound as an objective product require time and labor since the regioselectivity of the dehalogenation reaction is low and a 2-debrominated imidazole compound is produced in a large amount as a by-product.

Patent Document 1: JP-A-6-211846 (see Paragraphs 0105 to 0107)
Patent Document 2: WO00/06552 pamphlet (see Scheme 2 on page 4)
Patent Document 3: JP-A-2006-117628

### Disclosure of the Invention

### Problems that the Invention is to Solve

An object of the invention is to provide a process for producing a 2-haloimidazole compound represented by the general formula (II) of chem 4, which uses inexpensive raw materials and can produce it by simple and convenient operations at low costs.

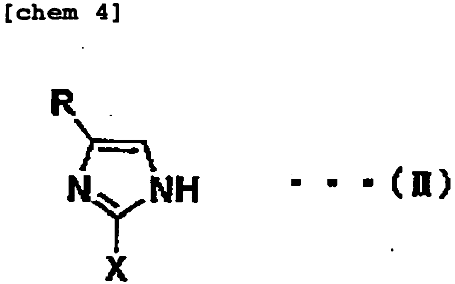

wherein R represents a hydrogen atom, an alkyl group, an aryl group, a nitro group, a cyano group, a hydroxyalkyl group, a formyl group, or a carboxyl group and X is a halogen atom.

### Means for Solving the Problems

As a result of the extensive studies for solving the above problems, the present inventors have found that the desired object can be achieved by reacting a 2,4-dihaloimidazole compound represented by the formula (I) of chem 5, an iodine compound, and one or two or more compounds selected from a ketone compound, an ester compound, and a nitrile compound. Thus, they have accomplished the invention.

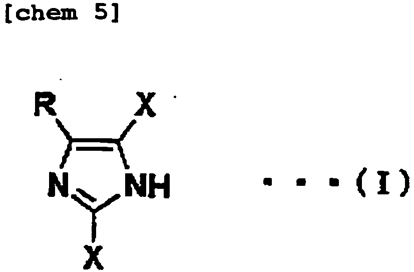

wherein R represents a hydrogen atom, an alkyl group, an aryl group, a nitro group, a cyano group, a hydroxyalkyl group, a formyl group, or a carboxyl group and X is a halogen atom.

### Advantage of the Invention

According to the production process of the invention, the 2-haloimidazole compound useful as an intermediate for medicaments and agricultural chemicals, an intermediate for dyes, and the like can be produced by a one-step reaction in a high yield and in a high purity, so that the production costs of the imidazole compound can be lowered.

### Best Mode for Carrying Out the Invention

The following will describe the invention in detail. The process for producing a 2-haloimidazole compound of the invention is characterized by reacting a 2,4-dihaloimidazole compound represented by the formula (I), an iodine compound, and one or two or more compounds selected from a ketone compound, an ester compound, and a nitrile compound. The above reaction is usually carried out in a solvent but, in the case where the raw material is liquid, it is possible to carry out the reaction without any solvent.

The 2,4-dihaloimidazole compound to be used in carrying out the invention can be synthesized by known methods (see, e.g., J. Chem. Soc., vol. 121, page 947 (1922)).

Examples of the 2,4-dihaloimidazole compound include:
2,4-dichloroimidazole,
2,4-dichloro-5-methylimidazole,
2,4-dichloro-5-ethylimidazole,
2,4-dichloro-5-propylimidazole,
4-butyl-2,5-dichloroimidazole,
2,4-dichloro-5-pentylimidazole,
2,4-dichloro-5-hexylimidazole,
2,4-dichloro-5-phenylimidazole,
2,4-dichloro-5-naphthylimidazole,
2,4-dichloro-5-nitroimidazole,
4-cyano-2,5-dichloroimidazole,
2,4-dichloro-5-hydroxymethylimidazole,
2,4-dichloro-5-hydroxyethylimidazole,
2,4-dichloro-5-formylimidazole,
2,5-dichloroimidazole-4-carboxylic acid,
2,4-dibromoimidazole,
2,4-dibromo-5-methylimidazole,
2,4-dibromo-5-ethylimidazole,
2,4-dibromo-5-propylimidazole,
4-butyl-2,5-dibromoimidazole,
2,4-dibromo-5-pentylimidazole,
2,4-dibromo-5-hexylimidazole,
2,4-dibromo-5-phenylimidazole,
2,4-dibromo-5-naphthylimidazole,
2,4-dibromo-5-nitroimidazole,
4-cyano-2,5-dibromoimidazole,
2,4-dibromo-5-hydroxymethylimidazole,
2,4-dibromo-5-hydroxyethylimidazole,
2,4-dibromo-5-formylimidazole,
2,5-dibromoimidazole-4-carboxylic acid, and the like.

Also, examples of the imidazole compounds produced corresponding to the above 2,4-dihaloimidazole compounds include:
2-chloroimidazole,
2-chloro-4-methylimidazole,
2-chloro-4-ethylimidazole,
2-chloro-4-propylimidazole,
4-butyl-2-chloroimidazole,
2-chloro-4-pentylimidazole,
2-chloro-4-hexylimidazole,
2-chloro-4-phenylimidazole,
2-chloro-4-naphthylimidazole,
2-chloro-4-nitroimidazole,
2-chloro-4-cyanoimidazole,
2-chloro-4-hydroxymethylimidazole,
2-chloro-4-hydroxyethylimidazole,
2-chloro-4-formylimidazole,
2-chloroimidazole-4-carboxylic acid,
2-bromoimidazole,
2-bromo-4-methylimidazole,
2-bromo-4-ethylimidazole,
2-bromo-4-propylimidazole,
2-bromo-4-butylimidazole,
2-bromo-4-pentylimidazole,
2-bromo-4-hexylimidazole,
2-bromo-4-phenylimidazole,
2-bromo-4-naphthylimidazole,
2-bromo-4-nitroimidazole,
2-bromo-4-cyanoimidazole,
2-bromo-4-hydroxymethylimidazole,
2-bromo-4-hydroxyethylimidazole,
2-bromo-4-formylimidazole, and
2-bromoimidazole-4-carboxylic acid.

Examples of the iodine compound to be used in carrying out the invention include iodine, hydroiodic acid, lithium iodide, sodium iodide, potassium iodide, calcium iodide, copper iodide, zinc iodide, magnesium iodide, aluminum iodide, ammonium iodide, and hydroiodide salts such as triethylamine hydroiodide. It is also possible to use them singly or in combination.
In order to increase the reaction yield, the amount of the above iodine compound may be sufficiently an excess amount, preferably 1.5 times equivalents or more relative to the 2,4-dihaloimidazole compound.

Examples of the ketone compound to be used in carrying out the invention include propanone, 2-butanone, 2-hexanone, 3-hexanone, cyclopentanone, cyclohexanone, cycloheptanone, and the like, and 2-hexanone, cyclopentanone, or cyclohexanone is preferable.

Moreover, examples of the ester compound to be used in carrying out the invention include ethyl acetate, butyl acetate, cyclohexyl acetate, ethyl butyrate, butyl butyrate, ethyl benzoate, butyl benzoate, diethyl malonate, diisopropyl malonate, and the like, and ethyl acetate, diethyl malonate, or diisopropyl malonate is preferable.

Furthermore, examples of the nitrile compound to be used in carrying out the invention include acetonitrile, butyronitrile, isobutyronitrile, hexanenitrile, cyclohexanecarbonitrile, acetophenyl cyanide, and the like, and butyronitrile, hexanenitrile, or cyclohexanecarbonitrile is preferable.

The amount of each of the above ketone compound, ester compound, and nitrile compound to be used may be sufficiently an excess amount, preferably 1.5 times equivalents or more relative to the 2,4-dihaloimidazole compound. In the case where these compounds are used in combination, the total amount of them may be sufficiently an excess amount, preferably 1.5 times equivalents or more relative to the 2,4-dihaloimidazole compound.

The solvent to be used in carrying out the invention is not particularly limited as long as it does not inhibit the reaction. Examples thereof include water, alcohols such as methanol, ethanol, and isopropyl alcohol; aliphatic hydrocarbons such as hexane and heptane; aromatic hydrocarbons such as benzene, toluene, and xylene; halogenated hydrocarbons such as methylene chloride, chloroform, carbon tetrachloride, chlorotrifluoromethane, dichloroethane, chlorobenzene, and dichlorobenzene; ethers such as diethyl ether, diisopropyl ether, tetrahydrofuran, dioxane, dimethoxyethane, and diethylene glycol dimethyl ether; amides such as formamide, N,N-dimethylformamide, N,N-dimethylacetamide, N-methyl-2-pyrrolidone, N-methylpyrrolidinone, and hexamethylphosphoric triamide; sulfoxides such as dimethyl sulfoxide; and the like. They can be used singly or in combination.

The reaction of the 2,4-dihaloimidazole compound and the iodine compound with the ketone compound, ester compound, or nitrile compound is carried out at a reaction temperature of 0 to 150°C, preferably 60 to 110°C and is completed for a reaction time of about 30 minutes to about 48 hours.
After completion of the reaction, the 2-haloimidazole compound as an objective product can be suitably isolated by an extraction operation with a solvent, a precipitation operation by cooling, or the like operation.
If necessary, the above 2-haloimidazole compound can be further purified by a treatment with active carbon, a recrystallization operation, and the like.
Incidentally, when the 2-chloroimidazole compound or 2-bromoimidazole compound is produced by carrying out the invention, a small amount of a 2-iodoimidazole compound is produced as a by-product together with the imidazole compounds. However, the 2-iodoimidazole compound is equivalent to the 2-chloroimidazole compound or 2-bromoimidazole compound and can be used as an intermediate for medicaments and agricultural chemicals, an intermediate for dyes, and the like without trouble even when the 2-iodoimidazole compound is contained in the 2-chloroimidazole compound or 2-bromoimidazole compound obtained in carrying out the invention.

### Examples

The following will specifically describe the invention with reference to Examples and Comparative Examples but it should not be construed that the invention is limited thereto.

### [Example 1]

While a mixture of 23.9 g (100.0 mmol) of 2,4-dibromo-5-methylimidazole and 30.0 g (300.0 mmol) of 2-hexanone was stirred at ordinary temperature, 22.5 g (150.0 mmol) of sodium iodide was added thereto to prepare a reaction liquid. After the reaction liquid was stirred at 105°C for 5 hours under a nitrogen stream, the liquid was stirred under ice cooling for 1 hour. When 2-bromo-4-methylimidazole in the reaction liquid was analyzed by means of HPLC, it was confirmed that the product was formed in an amount of 11.6 g (72.0 mmol, conversion: 72.0%).

After the reaction liquid was concentrated under reduced pressure, the product was purified by silica gel chromatography (ethyl acetate/hexane system) and, after volatile matter was further vaporized to solidify the product, crystals of 2-bromo-4-methylimidazole were obtained in an amount of 10.6 g (yield: 65.6%) as a residue.

Incidentally, the NMR spectrum of the crystals was measured and was compared with the NMR spectrum of its specimen, whereby it was confirmed that the product is 2-bromo-4-methylimidazole.

### [Example 2]

While a mixture of 23.9 g (100.0 mmol) of 2,4-dibromo-5-methylimidazole and 34.8 g (300.0 mmol) of butyl acetate was stirred at ordinary temperature, 22.5 g (150.0 mmol) of sodium iodide was added thereto to prepare a reaction liquid. After the reaction liquid was stirred at 105°C for 5 hours under a nitrogen stream, the liquid was stirred under ice cooling for 1 hour. When 2-bromo-4-methylimidazole in the reaction liquid was analyzed by means of HPLC, it was confirmed that the product was formed in an amount of 11.1 g (69.2 mmol, conversion: 69.2%).

After the reaction liquid was concentrated under reduced pressure, the product was purified by silica gel chromatography (ethyl acetate/hexane system) and, after volatile matter was further vaporized to solidify the product, crystals of 2-bromo-4-methylimidazole were obtained in an amount of 10.2 g (yield: 63.4%) as a residue.

Incidentally, the NMR spectrum of the crystals was measured and was compared with the NMR spectrum of its specimen, whereby it was confirmed that the product is 2-bromo-4-methylimidazole.

### [Example 3]

While a mixture of 23.9 g (100.0 mmol) of 2,4-dibromo-5-methylimidazole and 32.8 g (300.0 mmol) of cyclohexanecarbonitrile was stirred at ordinary temperature, 22.5 g (150.0 mmol) of sodium iodide was added thereto to prepare a reaction liquid. After the reaction liquid was stirred at 105°C for 5 hours under a nitrogen stream, the liquid was stirred under ice cooling for 1 hour. When 2-bromo-4-methylimidazole in the reaction liquid was analyzed by means of HPLC, it was confirmed that the product was formed in an amount of 9.7 g (60.2 mmol, conversion: 60.2%).

After the reaction liquid was concentrated under reduced pressure, the product was purified by silica gel chromatography (ethyl acetate/hexane system) and, after volatile matter was further vaporized to solidify the product, crystals of 2-bromo-4-methylimidazole were obtained in an amount of 8.4 g (yield: 52.4%) as a residue.

Incidentally, the NMR spectrum of the crystals was measured and was compared with the NMR spectrum of its specimen, whereby it was confirmed that the product is 2-bromo-4-methylimidazole.

### [Example 4]

While a mixture of 23.9 g (100.0 mmol) of 2,4-dibromo-5-methylimidazole and 48.1 g (300.0 mmol) of diethyl malonate was stirred at ordinary temperature, 4.5 g (150.0 mmol) of lithium iodide was added thereto to prepare a reaction liquid. After the reaction liquid was stirred at 105°C for 5 hours under a nitrogen stream, the liquid was stirred under ice cooling for 1 hour. When 2-bromo-4-methylimidazole in the reaction liquid was analyzed by means of HPLC, it was confirmed that the product was formed in an amount of 9.69 g (60.2 mmol, conversion: 60.2%).

After the reaction liquid was concentrated under reduced pressure, the product was purified by silica gel chromatography (ethyl acetate/hexane system) and, after volatile matter was further vaporized to solidify the product, crystals of 2-bromo-4-methylimidazole were obtained in an amount of 8.3 g (yield: 51.4%) as a residue.

Incidentally, the NMR spectrum of the crystals was measured and was compared with the NMR spectrum of its specimen, whereby it was confirmed that the product is 2-bromo-4-methylimidazole.

### [Example 5]

While a mixture of 23.9 g (100.0 mmol) of 2,4-dibromo-5-methylimidazole and 20.7 g (300.0 mmol) of butyronitrile was stirred at ordinary temperature, 22.0 g (75.0 mmol) of calcium iodide was added thereto to prepare a reaction liquid. After the reaction liquid was stirred at 105°C for 5 hours under a nitrogen steam, the liquid was stirred under ice cooling for 1 hour. When 2-bromo-4-methylimidazole in the reaction liquid was analyzed by means of HPLC, it was confirmed that the product was formed in an amount of 10.2 g (63.6 mmol, conversion: 63.6%).

After the reaction liquid was concentrated under reduced pressure, the product was purified by silica gel chromatography (ethyl acetate/hexane system) and, after volatile matter was further vaporized to solidify the product, crystals of 2-bromo-4-methylimidazole were obtained in an amount of 9.4 g (yield: 58.6%) as a residue.

Incidentally, the NMR spectrum of the crystals was measured and was compared with the NMR spectrum of its specimen, whereby it was confirmed that the product is 2-bromo-4-methylimidazole.

### [Example 6]

While a mixture of 23.9 g (100.0 mmol) of 2,4-dibromo-5-methylimidazole, 29.4 g (300.0 mmol) of cyclohexanone, and 23.9 g (259.4 mmol) of toluene was stirred at ordinary temperature, 22.5 g (150.0 mmol) of sodium iodide was added thereto to prepare a reaction liquid. After the reaction liquid was stirred at 105°C for 12 hours under a nitrogen stream, the liquid was stirred under ice cooling for 1 hour. When 2-bromo-4-methylimidazole in the reaction liquid was analyzed by means of HPLC, it was confirmed that the product was formed in an amount of 13.7 g (82.3 mmol, conversion: 85.3%).

After the reaction liquid was concentrated under reduced pressure, the product was purified by silica gel chromatography (ethyl acetate/hexane system) and, after volatile matter was further vaporized to solidify the product, crystals of 2-bromo-4-methylimidazole were obtained in an amount of 12.6 g (yield: 78.1%) as a residue.

Incidentally, the NMR spectrum of the crystals was measured and was compared with the NMR spectrum of its specimen, whereby it was confirmed that the product is 2-bromo-4-methylimidazole.

### [Example 7]

While a mixture of 23.9 g (100.0 mmol) of 2,4-dibromo-5-methylimidazole, 25.2 g (300.0 mmol) of cyclopentanone, and 29.1 g (300.0 mmol) of hexanenitrile was stirred at ordinary temperature, 22.5 g (150.0 mmol) of sodium iodide was added thereto to prepare a reaction liquid. After the reaction liquid was stirred at 105°C for 5 hours under a nitrogen stream, the liquid was stirred under ice cooling for 1 hour. When 2-bromo-4-methylimidazole in the reaction liquid was analyzed by means of HPLC, it was confirmed that the product was formed in an amount of 13.9 g (86.5 mmol, conversion: 86.5%).

After the reaction liquid was concentrated under reduced pressure, the product was purified by silica gel chromatography (ethyl acetate/hexane system) and, after volatile matter was further vaporized to solidify the product, crystals of 2-bromo-4-methylimidazole were obtained in an amount of 12.4 g (yield: 77.3%) as a residue.

Incidentally, the NMR spectrum of the crystals was measured and was compared with the NMR spectrum of its specimen, whereby it was confirmed that the product is 2-bromo-4-methylimidazole.

### [Example 8]

While a mixture of 30.2 g (100.0 mmol) of 2,4-dibromo-5-phenylimidazole and 30.0 g (300.0 mmol) of 2-hexanone was stirred at ordinary temperature, 22.5 g (150.0 mmol) of sodium iodide was added thereto to prepare a reaction liquid. After the reaction liquid was stirred at 105°C for 12 hours under a nitrogen stream, the liquid was stirred under ice cooling for 1 hour. When 2-bromo-4-phenylimidazole in the reaction liquid was analyzed by means of HPLC, it was confirmed that the product was formed in an amount of 13.4 g (60.0 mmol, conversion: 60.0%).

After the reaction liquid was concentrated under reduced pressure, the product was purified by silica gel chromatography (ethyl acetate/hexane system) and, after volatile matter was further vaporized to solidify the product, crystals of 2-bromo-4-phenylimidazole were obtained in an amount of 12.4 g (yield: 55.4%) as a residue.

Incidentally, the NMR spectrum of the crystals was measured and was compared with the NMR spectrum of its specimen, whereby it was confirmed that the product is 2-bromo-4-phenylimidazole.

### [Example 9]

While a mixture of 27.9 g (100.0 mmol) of 2,4-dibromo-5-nitroimidazole and 20.7 g (300.0 mmol) of isobutyronitrile was stirred at ordinary temperature, 22.5 g (150.0 mmol) of sodium iodide was added thereto to prepare a reaction liquid. After the reaction liquid was stirred at 105°C for 10 hours under a nitrogen stream, the liquid was stirred under ice cooling for 1 hour. When 2-bromo-4-nitroimidazole in the reaction liquid was analyzed by means of HPLC, it was confirmed that the product was formed in an amount of 12.4 g (64.6 mmol, conversion: 64.6%).

After the reaction liquid was concentrated under reduced pressure, the product was purified by silica gel chromatography (ethyl acetate/hexane system) and, after volatile matter was further vaporized to solidify the product, crystals of 2-bromo-4-nitroimidazole were obtained in an amount of 11.6 g (yield: 60.6%) as a residue.

Incidentally, the NMR spectrum of the crystals was measured and was compared with the NMR spectrum of its specimen, whereby it was confirmed that the product is 2-bromo-4-nitroimidazole.

### [Example 10]

While a mixture of 25.1 g (100.0 mmol) of 4-cyano-2,5-dibromoimidazole and 42.7 g (300.0 mmol) of cyclohexyl acetate was stirred at ordinary temperature, 22.5 g (150.0 mmol) of sodium iodide was added thereto to prepare a reaction liquid. After the reaction liquid was stirred at 105°C for 12 hours under a nitrogen stream, the liquid was stirred under ice cooling for 1 hour. When 2-bromo-4-cyanoimidazole in the reaction liquid was analyzed by means of HPLC, it was confirmed that the product was formed in an amount of 11.2 g (65.3 mmol, conversion: 65.3%).

After the reaction liquid was concentrated under reduced pressure, the product was purified by silica gel chromatography (ethyl acetate/hexane system) and, after volatile matter was further vaporized to solidify the product, crystals of 2-bromo-4-cyanoimidazole were obtained in an amount of 10.5 g (yield: 61.2%) as a residue.

Incidentally, the NMR spectrum of the crystals was measured and was compared with the NMR spectrum of its specimen, whereby it was confirmed that the product is 2-bromo-4-cyanoimidazole.

### [Example 11]

While a mixture of 16.5 g (100.0 mmol) of 2,4-dichloro-5-ethylimidazole and 30.0 g (300.0 mmol) of 3-hexanone was stirred at ordinary temperature, 22.5 g (150.0 mmol) of sodium iodide was added thereto to prepare a reaction liquid. After the reaction liquid was stirred at 105°C for 12 hours under a nitrogen stream, the liquid was stirred under ice cooling for 1 hour. When 2-chloro-4-ethylimidazole in the reaction liquid was analyzed by means of HPLC, it was confirmed that the product was formed in an amount of 8.3 g (63.6 mmol, conversion: 63.6%).

After the reaction liquid was concentrated under reduced pressure, the product was purified by silica gel chromatography (ethyl acetate/hexane system) and, after volatile matter was further vaporized to solidify the product, crystals of 2-chloro-4-ethylimidazole were obtained in an amount of 7.6 g (yield: 58.3%) as a residue.

Incidentally, the NMR spectrum of the crystals was measured and was compared with the NMR spectrum of its specimen, whereby it was confirmed that the product is 2-chloro-4-ethylimidazole.

### [Example 12]

While a mixture of 16.7 g (100.0 mmol) of 2,4-dichloro-5-hydroxymethylimidazole and 56.5 g (300.0 mmol) of diisopropyl malonate was stirred at ordinary temperature, 22.5 g (150.0 mmol) of sodium iodide was added thereto to prepare a reaction liquid. After the reaction liquid was stirred at 105°C for 12 hours under a nitrogen stream, the liquid was stirred under ice cooling for 1 hour. When 2-chloro-4-hydroxymethylimidazole in the reaction liquid was analyzed by means of HPLC, it was confirmed that the product was formed in an amount of 6.6 g (45.7 mmol, conversion: 45.7%).

After the reaction liquid was concentrated under reduced pressure, the product was purified by silica gel chromatography (ethyl acetate/hexane system) and, after volatile matter was further vaporized to solidify the product, crystals of 2-chloro-4-hydroxymethylimidazole were obtained in an amount of 5.3 g (yield: 40.1%) as a residue.

Incidentally, the NMR spectrum of the crystals was measured and was compared with the NMR spectrum of its specimen, whereby it was confirmed that the product is 2-chloro-4-hydroxymethylimidazole.

### [Example 13]

While a mixture of 16.5 g (100.0 mmol) of 2,4-dichloro-5-formylimidazole and 29.4 g (300.0 mmol) of cyclohexanone was stirred at ordinary temperature, 22.5 g (150.0 mmol) of sodium iodide was added thereto to prepare a reaction liquid. After the reaction liquid was stirred at 105°C for 12 hours under a nitrogen stream, the liquid was stirred under ice cooling for 1 hour. When 2-chloro-4-formylimidazole in the reaction liquid was analyzed by means of HPLC, it was confirmed that the product was formed in an amount of 9.3 g (70.9 mmol, conversion: 70.9%).

After the reaction liquid was concentrated under reduced pressure, the product was purified by silica gel chromatography (ethyl acetate/hexane system) and, after volatile matter was further vaporized to solidify the product, crystals of 2-chloro-4-formylimidazole were obtained in an amount of 8.6 g (yield: 66.1%) as a residue.

Incidentally, the NMR spectrum of the crystals was measured and was compared with the NMR spectrum of its specimen, whereby it was confirmed that the product is 2-chloro-4-formylimidazole.

### [Example 14]

While a mixture of 18.1 g (100.0 mmol) of 2,5-dichloroimidazole-4-carboxylic acid and 32.8 g (300.0 mmol) of cyclohexanecarbonitrile was stirred at ordinary temperature, 22.5 g (150.0 mmol) of sodium iodide was added thereto to prepare a reaction liquid. After the reaction liquid was stirred at 105°C for 12 hours under a nitrogen stream, the liquid was stirred under ice cooling for 1 hour. When 2-chloroimidazole-4-carboxylic acid in the reaction liquid was analyzed by means of HPLC, it was confirmed that the product was formed in an amount of 7.4 g (50.2 mmol, conversion: 50.2%).

After the reaction liquid was concentrated under reduced pressure, the product was purified by silica gel chromatography (ethyl acetate/hexane system) and, after volatile matter was further vaporized to solidify the product, crystals of 2-chloroimidazole-4-carboxylic acid were obtained in an amount of 6.8 g (yield: 46.5%) as a residue.

Incidentally, the NMR spectrum of the crystals was measured and was compared with the NMR spectrum of its specimen, whereby it was confirmed that the product is 2-chloroimidazole-4-carboxylic acid.

### [Comparative Example 1]

While a solution of 24.0 g (100.0 mmol) of 2,4-dibromo-5-methylimidazole in 120 ml of ethanol was stirred at ordinary temperature, 22.5 g (150.0 mmol) of sodium iodide was added thereto to prepare a reaction liquid. After the reaction liquid was stirred at 105°C for 5 hours under a nitrogen stream, the formation of 2-bromo-4-methylimidazole was not observed when the reaction liquid was analyzed by means of HPLC.

### [Comparative Example 2]

While a mixture of 24.0 g (100.0 mmol) of 2,4-dibromo-5-methylimidazole and 30.0 g (300.0 mmol) of 2-hexanone was stirred at ordinary temperature, 15.4 g (150.0 mmol) of sodium bromide was added thereto to prepare a reaction liquid. After the reaction liquid was stirred at 105°C for 5 hours under a nitrogen stream, the formation of 2-bromo-4-methylimidazole was not observed when the reaction liquid was analyzed by means of HPLC.

While the invention has been described in detail and with reference to specific embodiments thereof, it will be apparent to one skilled in the art that various changes and modifications can be made therein without departing from the spirit and scope thereof.
The present application is based on Japanese Patent Application No. 2007-097005 filed on April 3, 2007 and Japanese Patent Application No. 2008-073511 filed on March 21, 2008, and the contents are incorporated herein by reference.

### Industrial Applicability

According to the production process of the invention, the 2-haloimidazole compound useful as an intermediate for medicaments and agricultural chemicals, an intermediate for dyes, and the like can be produced by a one-step reaction in a high yield and in a high purity, so that the production costs of the imidazole compound can be lowered.

## Claims

1. A process for producing a 2-haloimidazole compound represented by the formula (II), which comprises reacting a 2,5-dihaloimidazole compound represented by the formula (I), an iodine compound, and one or two or more compounds selected from a ketone compound, an ester compound, and a nitrile compound: 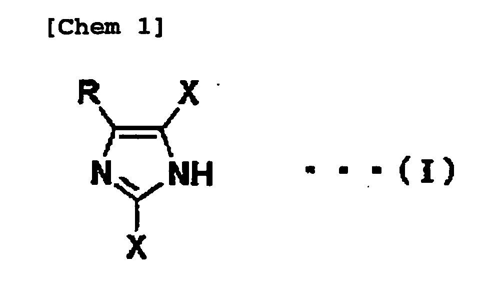 wherein R represents a hydrogen atom, an alkyl group, an aryl group, a nitro group, a cyano group, a hydroxyalkyl group, a formyl group, or a carboxyl group and X is a halogen atom. 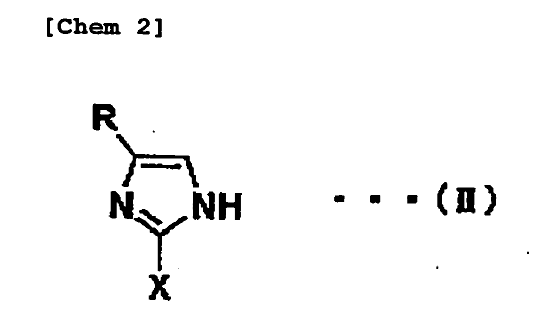 wherein R represents a hydrogen atom, an alkyl group, an aryl group, a nitro group, a cyano group, a hydroxyalkyl group, a formyl group, or a carboxyl group and X is a halogen atom.

2. The process for producing a 2-haloimidazole compound according to claim 1, wherein the iodine compound is iodine, hydroiodic acid, lithium iodide, sodium iodide, potassium iodide, calcium iodide, copper iodide, zinc iodide, magnesium iodide, aluminum iodide, ammonium iodide, or triethylamine hydroiodide.

3. The process for producing a 2-haloimidazole compound according to claim 1, wherein the ketone compound is propanone, 2-butanone, 2-hexanone, 3-hexanone, cyclopentanone, cyclohexanone, or cycloheptanone; the ester compound is ethyl acetate, butyl acetate,
cyclohexyl acetate, ethyl butyrate, butyl butyrate, ethyl benzoate, butyl benzoate, diethyl malonate, or diisopropyl malonate; the nitrile compound is acetonitrile, butyronitrile, isobutyronitrile, hexanenitrile, cyclohexanecarbonitrile, or acetophenyl cyanide.

## Patentansprüche

1. Verfahren zum Herstellen einer 2-Haloimidazol-Verbindung, die durch die Formel (II) dargestellt wird, wobei das Verfahren umfasst, eine 2,5-Dihaloimidazol-Verbindung, die durch die Formel (I) dargestellt wird, eine Iodverbindung und eine oder zwei oder mehr Verbindungen, die unter einer Ketonverbindung, einer Esterverbindung und einer Nitrilverbindung ausgewählt wurden, miteinander zur Reaktion zu bringen: 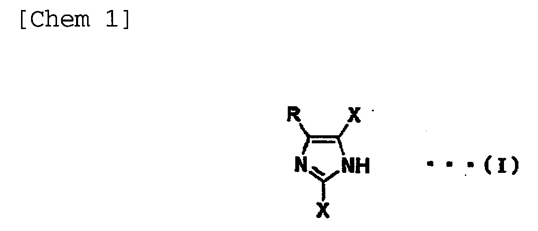 wobei R ein Wasserstoffatom, eine Alkyl-Gruppe, eine Acryl-Gruppe, eine Nitro-Gruppe, eine Cyano-Gruppe, eine Hydroxyalkyl-Gruppe, eine Formyl-Gruppe oder eine Carboxyl-Gruppe darstellt und X ein Halogenatom ist, 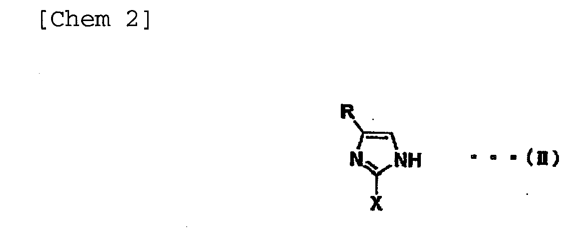 wobei R ein Wasserstoffatom, eine Alkyd-Gruppe, eine Aryl-Gruppe, eine Nitro-Gruppe, eine Cyano-Gruppe, eine Hydroxyalkyl-Gruppe, eine Formyl-Gruppe oder eine Carboxyl-Gruppe darstellt und X ein Halogenatom ist.

2. Verfahren zum Herstellen einer 2-Haloimidazol-Verbindung nach Anspruch 1, wobei die Iodverbindung Iod, Iodwasserstoffsäure, Lithiumiodid, Natriumiodid, Kaliumiodid, Kalziumiodid, Kupferiodid, Zinkiodid, Magnesiumiodid, Aluminiumiodid, Ammoniumiodid oder Triethylamin-Hydroiodid ist.

3. Verfahren zum Herstellen einer 2-Haloimidazol-Verbindung nach Anspruch 1, wobei die Ketonverbindung Propanon, 2-Butanon, 2-Hexanon, 3-Hexanon, Cyclopentanon, Cyclohexanon oder Cycloheptanon ist; die Esterverbindung Ethylacetat, Butylacetat, Cyclohexylacetat, Ethylbutyrat, Butylbutyrat, Ethylbenzoat, Butylbenzoat, Diethylmalonat oder Diisopropylmalonat ist; und die Nitrilverbindung Acetonitril, Butyronitril, Isobutyronitril, Hexanenitril, Cyclohexancarbonitril oder Acetophenylcyanid ist.

## Revendications

1. Procédé pour produire un composé 2-haloimidazole représenté par la formule (II), qui comprend la mise à réagir d'un composé 2,5-dihaloimidazole représenté par la formule (I), un composé iodé, et un ou deux autres composés choisis parmi un groupe cétone, un groupe ester et un groupe nitrile : 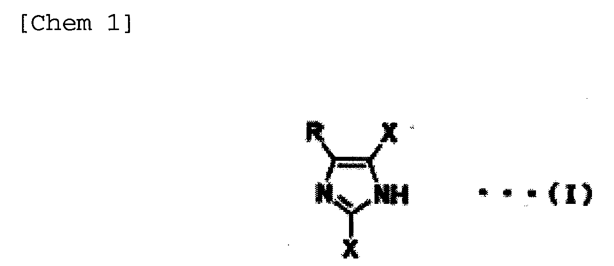 dans laquelle R représente un atome d'hydrogène, un groupe alkyle, un groupe aryle, un groupe nitro, un groupe cyano, un groupe hydroxyalkyle, un groupe formyle ou un groupe carboxyle et X est un atome d'halogène, 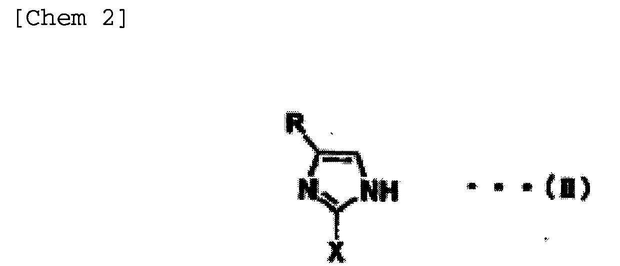 dans laquelle R représente un atome d'hydrogène, un groupe alkyle, un groupe aryle, un groupe nitro, un groupe cyano, un groupe hydroxyalkyle, un groupe formyle ou un groupe carboxyle et X est un atome d'halogène.

2. Procédé pour produire un composé 2-haloimidazole selon la revendication 1, dans lequel le composé iodé est l'iode, l'acide hydroïodique, l'iodure de lithium, l'iodure de sodium, l'iodure de potassium, l'iodure de calcium, l'iodure de cuivre, l'iodure de zinc, l'iodure de magnésium, l'iodure d'aluminium, l'iodure d'ammonium ou l'hydroïodure de triéthylamine.

3. Procédé pour produire un composé 2-haloimidazole selon la revendication 1, dans lequel le composé cétone est la propanone, la 2-butanone, la 2-hexanone, la 3-hexanone, la cyclopentanone, la cyclohexanone ou la cycloheptanone ; le composé ester est l'acétate d'éthyle, l'acétate de butyle, l'acétate de cyclohexyle, le butyrate d'éthyle, le butyrate de butyle, le benzoate d'éthyle, le benzoate de butyle, le malonate de diéthyle ou le malonate de diisopropyle ; le composé nitrile est l'acétonitrile, le butyronitrile, l'isobutyronitrile, l'hexanenitrile, le cyclohexanecarbonitrile ou le cyanure d'acétophényle.
